# EUROPEAN PATENT SPECIFICATION

(11) **EP 1 452 169 B1**
(45) Date of publication and mention of the grant of the patent: **24.01.2007**
(21) Application number: 02793140.1
(22) Date of filing: 02.12.2002
(51) Int. Cl.: A61K 9/10, A61K 31/192

(54) **AQUEOUS BASE LIQUID PHARMACEUTICAL COMPOSITIONS IN SUSPENSION FORM FOR THE ORAL ADMINISTRATION OF IBUPROFEN**
FLÜSSIGE PHARMAZEUTISCHE ZUSAMMENSETZUNGEN AUF WASSERBASIS IN SUSPENSIONSFORM ZUR ORALEN VERABREICHUNG VON IBUPROFEN
COMPOSITIONS PHARMACEUTIQUES LIQUIDES DE BASE AQUEUSE EN FORME DE SUSPENSION DESTINNEES A L'ADMINISTRATION PAR VOIE ORALE D'IBUPROFENE

(30) Priority: 04.12.2001 ES 200102705
(43) Date of publication of application: 01.09.2004
(73) Proprietor: Farmalider, S.A., E-28100 Alcobendas (Madrid) (ES)
(72) Inventor: JIMENEZ REDONDO, Ana, Maria, E-28100 Alcobendas (Madrid) (ES); BERGES ABANADES, Laura, E-28100 Alcobendas (Madrid) (ES)
(74) Representative: Gil-Vega, Victor
(86) International application number: PCT/ES2002/000572
(87) International publication number: WO 2003/047550

(56) References cited:
- EP-A- 0 684 819
- ES-T- 2 039 281
- ES-T- 2 048 428
- ES-T- 2 064 634
- US-B1- 6 231 890

## Description

This invention relates to new aqueous-based liquid pharmaceutical compositions in suspension form for the oral administration of ibuprofen as the active principle which comprises 0.8% to 6% in weight per volume of sodium chloride, from 0.1% to 2% in weight per volume of sodium chloride; from 0.01% to 0.5% in weight per volume of sodium saccharin; from 0.1% to 2% in weight per volume of one or more pH-regulating agents in sufficient quantities to adjust the pH to between 3.8 and 4.8; from 0.01% to 0.5 % in weight per volume of a preservative; together with an suitable quantity of other pharmaceutically acceptable excipients with viscosifying and taste-masking characteristics, as well as the process for its preparation.

### BACKGROUND OF THE INVENTION

A large number of different classes of substances belong to the non-steroidal group of antirheumatics, to which the acid character of the different active substances, amongst which ibuprofen is found, is predominantly common.

2-(4-isobutylphenyl) propionic acid, known under the name of ibuprofen, is a generally well-tolerated active principle, with analgesic, antipyretic and anti-inflammatory activities.

Ibuprofen has shown to be efficient by inhibiting the synthesis of prostaglandins, e.g. in inflammation models of animal experiments. In human therapy, ibuprofen reduces pain, swelling and fever caused by inflammation.

Various solutions in the state of the art have been proposed to prepare ibuprofen-based medicines for the treatment of joint pain, inflammation, fever, swelling from arthritis or gout, as a painkiller in the treatment of dysmenorrhea (painful or difficult menstruation) and the treatment of juvenile rheumatoid arthritis.

The bitter taste of ibuprofen is characteristic, as is the burning sensation in the throat after ingesting it.

Ibuprofen's bad taste can usually be masked by the presence of natural sugar, amongst other masking agents, for which reason the administration of said pharmaceutical compositions is usually unsuitable for diabetics or people who cannot take sugar.

Ibuprofen is essentially insoluble in water in the range of pH 1 to 4, showing a dramatic increase in solubility at pH 5; as human saliva normally has a pH ranging from 5.6 to 7.6, it is necessary for the pH of the suspension to be adjusted so that its buffering capacity inhibits the ibuprofen from dissolving in the human saliva, avoiding, in this manner, the subsequent characteristic bitter taste of ibuprofen.

In the preparation of some of these formulations ibuprofen is not used in the form of free acid, but in the form of a salt with an organic or inorganic base; thus, its lysine salt is used in ointments, injections, suppositories and tablets and its methylglucomine salt is used in ointments, injections and tablets and its sodium salt has been disclosed and commercialized for suppositories.

The patent US 4684666 discloses a liquid ibuprofen composition for oral administration in the form of a syrup where ibuprofen is suspended in an aqueous liquid with more than 50% in weight of a polyhydric alcohol thickening agent, a sweetening agent and a pH greater than 7.0 and less than 7.7.

The patent ES2048428 discloses a process for the preparation of an aqueous-based ibuprofen composition where microcrystalline cellulose, carboxymethylcellulose and polisorbate 80 are stabilizing agents of the suspension, and a buffering acid is present to attain a pH between 1.5 and 3.5 and a buffering capacity between 0.03 and 0.05 between the initial pH and a pH greater than the initial pH by 1.0 units.

The patent ES2039281 relates to an ibuprofen suspension for paediatric use, where xanthan gum, microcrystalline cellulose, carboxymethylcellulose and polisorbate 80 are stabilizing agents, sucrose and sorbitol are taste-masking agents and the pH ranges from 3.5 to 5.0.

Therefore, there exists the necessity to obtain a liquid pharmaceutical composition of ibuprofen in the form of a suspension, without sugars for it to be administered to diabetic patients or to those who cannot take sugar.

### DESCRIPTION OF THE INVENTION

This invention resolves the aforementioned problems, providing a new aqueous-based liquid pharmaceutical composition in the form of a suspension for the oral administration of ibuprofen as the active principle which comprises 0.8% to 6% in weight per volume of ibuprofen; from 0.1% to 2% in weight per volume of sodium chloride; from 0.01% to 0.5% in weight per volume of sodium saccharin; from 0.1% to 2% in weight per volume of one or more pH-regulating agents in sufficient quantities to adjust the pH to between 3.8 and 4.8; from 0.01% to 0.5% in weight per volume of a preservative selected from the group consisting of sodium benzoate, benzoic acid, potassium sorbate, sorbic acid, methyl p-hydroxibenzoate, ethyl p-hydroxibenzoate, propyl p-hydroxibenzoate, butyl p-hydroxibenzoate, sodium methyl p-hydroxibenzoate, sodium ethyl p-hydroxibenzoate, sodium propyl p-hydroxibenzoate, sodium butyl p-hydroxibenzoate, domiphen bromide, sodium propionate, propylene glycol and their mixtures; together with an suitable quantity of other pharmaceutically acceptable excipients with viscosifying and taste-masking qualities.

In a preferred embodiment of this invention the pH-regulating agent is selected from the group consisting of anhydrous citric acid, monohydrate citric acid, lactic acid, tartaric acid, fumaric acid, malic acid, their corresponding alkaline salts and their mixtures.

In a further preferred embodiment, the pH-regulating agent is selected from the group consisting of anhydrous citric acid, monohydrate citric acid, sodium citrate, potassium citrate and their mixtures.

In another preferred embodiment of the invention, the quantity of preservative ranges from 0.1% to 0.2% in weight per volume and this is selected from the group consisting of benzoic acid, sodium benzoate, potassium sorbate, sorbic acid and their mixtures.

As it is a thermodynamically unstable dispersed system where the solid particles of the internal phase tend to aggregate and form sediment, it requires the use of coadjuvant agents to improve humidity, dispersion, viscosity and other aspects so that, acting on the characteristics and properties of both the particles and their interaction in the dispersal medium, good product stability is attained.

In a preferred embodiment of the invention, there is between 0.1% and 1% in weight per volume of a viscosifier selected from the group consisting of hypromellose, hydroxypropyl cellulose, hydroxiethyl cellulose, hydroxypropyl methylcellulose, xanthan gum, guar gum, tragacanth gum, bentonite, propylene glycol alginate, sodium alginate, povidone, magnesium and aluminium silicate, kaolin, gelatine, colloidal silica dioxide and their mixtures.

In a particularly preferred embodiment, the viscosifier is selected from the group consisting of xanthan gum, guar gum, hydroxipropyl cellulose, hydroxiethyl cellulose, hydroxypropyl methylcellulose and their mixtures.

In a preferred embodiment of this invention, there is an effective quantity of a wetting agent selected from the group consisting of glycerol, propylene glycol, liquid polyethylene glycols, sorbitol and their mixtures.

In addition to the presence of sodium saccharin and sodium chloride as taste-masking agents, in a preferred embodiment of this invention there is an effective quantity of a taste-masking agent selected from the group consisting of saccharin, thaumatin, maltitol syrup, acesulfame potassium, aspartame, maltitol, lactitol, sodium cyclamate, xylitol, sorbitol and their combinations.

In a particularly preferred embodiment of this invention, in addition to sodium saccharin and sodium chloride as taste-masking agents, there is an effective quantity of a taste-masking agent selected from the group consisting of saccharin, thaumatin, maltitol syrup, sorbitol and their mixtures.

In a further preferred embodiment, there is between 0.0005% and 0.010% in weight per volume of thaumatin as a taste-masking agent.

The size of the ibuprofen particles should be suitable to provide a suspension, i.e. not too fine to cause the ibuprofen to float and not too large to make them sink. The particle size also plays an important role in achieving the masking of the characteristically bitter taste of the ibuprofen, as the smaller the particle size the bigger the specific surface area and therefore the characteristic bitterness will be intensified. Typical average particle sizes should range from 30 to 250 µpm, the preferred particle size ranging from 30 to 50 µpm.

A further object of this invention is a process for the preparation of an aqueous-based liquid pharmaceutical composition starting from ibuprofen, according to that described above, which consists of:
a) preparing an aqueous solution with the preservative and pH regulators:
b) the addition of sodium saccharin, sodium chloride and other taste-masking agents;
c) the addition of the viscosifying agents;
d) wetting the active principle by incorporating a wetting agent;
e) the incorporation of an active wetting principle, obtained in section d), in the mixture obtained in section c);

### Step a)

A suitable amount of purified water is placed in a reactor and a preservative is added by stirring until it is completely dissolved.

The buffering acid is added, by stirring, to the above solution until it is completely dissolved and, next, the alkaline salt of a buffering acid is added without stopping the stirring.

### Step b)

The first sweetener (sodium saccharin) is added, by stirring, to the above solution until it is completely dissolved. The sodium chloride is subsequently added as flavouring, keeping stirring until it is completely dissolved.

In a separate reactor, place a suitable quantity of water in which the remaining taste-masking agents have been dissolved.

### Step c)

The viscosifying agents are slowly added to the above solution by stirring, maintaining this stirring for a minimum of 3.5 hours. Subsequently, it is left to rest for a minimum of 12 hours until the mixtures acquires consistency.

### Step d)

The wetting agent is placed in a third reactor and the active principle ibuprofen screened at that time, is added, constantly stirring.

### Step e)

The mixture from the third reactor, containing the appropriately wetted ibuprofen, is added to the mixture in the first reactor, stirring until homogenisation is achieved.

Next, a suitable quantity of water is added to reach the final size of the batch, stirring until homogeneity, for at least one hour.

It is known that the boiling point of ibuprofen is between 75°C and 77°C, and if the ibuprofen goes above or close to this temperature during the manufacturing phase of the pharmaceutical composition it can undergo degradation producing the degradation substances 2-(4-butylphenyl)-propionic acid or 4-isobutyl acetephenone acid.

In a preferred embodiment of this invention, the process for the preparation of the abovementioned pharmaceutical composition of ibuprofen is performed at room temperature.

The invention is illustrated with the following non-restrictive examples.

### EXAMPLES

### Example 1

### Preparation of a strawberry-flavoured ibuprofen suspension

According to the abovementioned preparation process, an ibuprofen suspension is prepared which contains 2% in weight per volume of the total composition of ibuprofen, whose qualitative composition was the following:

| Ingredients | %w/v |
|---|---|
| Ibuprofen | 2.0 g |
| Sodium benzoate | 0.1 g |
| Anhydrous citric acid | 0.5 g |
| Tribasic sodium citrate | 0.6 g |
| Sodium saccharin | 0.05 g |
| Sodium chloride | 0.5 g |
| Hydroxipropyl methylcellulose | 0.5 g |
| Xanthan gum | 0.4 g |
| Maltitol syrup 80/55 | 50.0 g |
| Strawberry flavour | 0.07 g |
| Azorubine colouring | 0.0015 g |
| Glycerine | 5.0 g |
| Purified Water | until 100 ml |

Obtaining, in this manner, a pink-coloured, viscose suspension, where the initial pH of the formulation was 4.2, with viscosity between 350 cps and 800 cps.

The formulation has a sweet, pleasant strawberry flavour with no discernable unpleasant taste, characteristic of ibuprofen, after tasting or swallowing it.

### Example 2

### Preparation of an orange-flavoured ibuprofen suspension

According to the abovementioned preparation process, an ibuprofen suspension is prepared which contains 2% in weight per volume of the total composition of ibuprofen, whose qualitative composition was the following:

| Ingredients | %w/v |
|---|---|
| Ibuprofen | 2.0 g |
| Sodium benzoate | 0.1 g |
| Anhydrous citric acid | 0.5 g |
| Sodium citrate | 0.6 g |
| Sodium saccharin | 0.05 g |
| Sodium chloride | 0.5 g |
| Maltitol syrup 80/55 | 50,0 g |
| Xanthan gum | 0,4 g |
| Hydroxipropyl methylcellulose | 0,5 g |
| Orange flavour | 0.05 g |
| Lemon flavour | 0,03 g |
| E-110 colouring | 0.02 g |
| Glycerine | 5.0 g |
| Purified Water | until 100 ml |

Obtaining, in this manner, an orange-coloured viscose suspension, where the initial pH of the formulation was 4.2, with viscosity between 350 cps and 800 cps.

The formulation has a sweet, pleasant orange flavour with no discernable unpleasant taste, characteristic of ibuprofen, after tasting or swallowing it.

### Example 3

### Preparation of a strawberry-flavoured ibuprofen suspension

According to the abovementioned preparation process, an ibuprofen suspension is prepared which contains 4% in weight per volume of the total composition of ibuprofen, whose qualitative composition was the following:

| Ingredients | %w/v |
|---|---|
| Ibuprofen | 4.0 g |
| Sodium benzoate | 0.1 g |
| Anhydrous citric acid | 0.6 g |
| Sodium citrate | 0.7 g |
| Sodium saccharin | 0.05 g |
| Sodium chloride | 1.0 g |
| Maltitol syrup 80/55 | 50.0 g |
| Xanthan gum | 0.4 g |
| Hypromellose | 0.5 g |
| Strawberry flavour | 0.07 g |
| Azorubine | 0.0015 g |
| Thaumatin | 0.001 g |
| Glycerine | 10.0 g |
| Purified Water | until 100 ml |

Obtaining, in this manner, a pink-coloured viscose suspension, where the initial pH of the formulation was 4.6, with viscosity between 350 cps and 800 cps.

The formulation has a sweet, pleasant strawberry flavour with no discernable unpleasant taste, characteristic of ibuprofen, after tasting or swallowing it.

## Claims

1. Aqueous-based liquid pharmaceutical composition in suspension form for the oral administration of ibuprofen as the active principle, **characterized in that** said composition contains:
a) from 0.8% to 6% in weight per volume of ibuprofen
b) from 0.1% to 2% in weight per volume of sodium chloride;
c) from 0.01% to 0.5% in weight per volume of sodium saccharin
d) from 0.1% to 2% in weight per volume of one or more pH-regulating agents in sufficient quantities to adjust the pH to between 3.8 and 4.8;
e) from 0.01% to 0.5% in weight per volume of a preservative selected from the group consisting of sodium benzoate, benzoic acid, potassium sorbate, sorbic acid, methyl p-hydroxibenzoate, ethyl p-hydroxibenzoate, propyl p-hydroxibenzoate, butyl p-hydroxibenzoate, sodium methyl p-hydroxibenzoate, sodium ethyl p-hydroxibenzoate, sodium propyl p-hydroxibenzoate, sodium butyl p-hydroxibenzoate, domiphen bromide, sodium propionate, propylene glycol and their mixtures;
together with a suitable quantity of other pharmaceutically acceptable excipients with viscosifying and taste-masking characteristics.

2. Composition as claimed in claim 1, **characterized in that** the pH-regulating agent is selected from the group consisting of anhydrous citric acid, monohydrate citric acid, lactic acid, tartaric acid, fumaric acid, malic acid, their corresponding alkaline salts and their mixtures.

3. Composition as claimed in claim 2, **characterized in that** the pH-regulating agent is selected from the group consisting of anhydrous citric acid, monohydrate citric acid, sodium citrate, potassium citrate and their mixtures.

4. Composition as claimed in any of claims 1 to 3, **characterized in that** the quantity of preservative ranges from 0.1% to 0.2% in weight per volume and this is selected from the group consisting of benzoic acid, sodium benzoate, potassium sorbate, sorbic acid and their mixtures.

5. Composition as claimed in any of the preceding claims, **characterized in that** there is between 0.1% and 1% in weight per volume, of a viscosifier selected from the group consisting of hypromellose, hydroxypropyl cellulose, hydroxiethyl cellulose, hydroxypropyl methylcellulose, xanthan gum, guar gum, tragacanth gum, bentonite, propylene glycol alginate, sodium alginate, povidone, magnesium and aluminium silicate, kaolin, gelatine, colloidal silica dioxide and their mixtures.

6. Composition as claimed in claim 5, **characterized in that** the viscosifier is selected from the group consisting of xanthan gum, guar gum, hydroxipropyl cellulose, hydroxiethyl cellulose, hydroxypropyl methylcellulose and their mixtures.

7. Composition as claimed in any of the preceding claims, **characterized in that** there is an effective quantity of a wetting agent selected from the group consisting of glycerol, propylene glycol, liquid polyethylene glycols, sorbitol and their mixtures.

8. Composition as claimed in any of the preceding claims, further **characterized in that** there is an effective quantity of a taste-masking agent selected from the group consisting of saccharin, thaumatin, maltitol syrup, acesulfame potassium, aspartame, maltitol, lactitol, sodium cyclamate, xylitol, sorbitol and their combinations.

9. Composition as claimed in any of the preceding claims, **characterized in that** there is between 0.0005% and 0.010 % in weight per volume of thaumatin as a taste-masking agent.

10. Process for the preparation of an aqueous-based liquid pharmaceutical composition in suspension form for the oral administration of ibuprofen as the active principle, **characterized in that** it consists of
a) preparing an aqueous solution with the preservative and pH regulators:
b) the addition of sodium saccharin, sodium chloride and other taste-masking agents;
c) the addition of viscosifying agents;
d) wetting the active principle by incorporating a wetting agent;
e) the incorporation of an active wetting principle, obtained in section d), in the mixture obtained in section c);

11. Process as claimed in claim 10, **characterized in that** all the components are added at room temperature.

12. Process as claimed in either claim 10 or 11, **characterized in that** the quantity of preservative added in step a) ranges from 0.1% to 0.2% in weight per volume.

13. Process as claimed in any of claims 10 to 12, **characterized in that** the quantity of sodium saccharin and sodium chloride added in step b) is between 0.01% and 0.5% and 0.1% and 2% in weight per volume, respectively.

14. Process as claimed in any of claims 10 to 13, **characterized in that** the quantity of pH-regulating agents is between 0.1% and 2% in weight per volume, so that the pH is adjusted to between 3.8 and 4.8.

## Patentansprüche

1. Flüssige, wässrige pharmazeutische Zusammensetzung in Form einer Suspension für orale Verabreichung von Ibuprofen als Wirkstoff, **dadurch gekennzeichnet, dass** diese Zusammensetzung folgendes enthält:
a) Ibuprofen o,8 bis 6 Gew.-% pro Volumen;
b) Natriumchlorid 0,1 bis 2 Gew.-% pro Volumen;
c) Natriumsaccharin 0,01 bis 0,5 Gew.-% pro Volumen;
d) 0,1 bis 2 Gew.-% pro Volumen eines oder mehrerer pH-Regulierungsmittel in ausreichenden Mengen zur Einstellung des pH-Wertes zwischen 3,8 und 4,8;
e) 0,01 bis 0,5 Gew.-% pro Volumen eines Konsiervierungsmittels ausgewählt aus der Gruppe Natriumbenzoat, Benzoesäure, Kaliumsorbat, Sorbinsäure, Methyl p-Hydroxibenzoat, Ethyl p-Hydroxibenzoat, Propyl p-Hydroxibenzoat, Butyl p-Hydroxibenzoat, Natrium Methyl-p-Hydroxibenzoat, Natrium Ethyl p-Hydroxibenzoat, Natrium Propyl p-Hydroxibenzoat, Natrium Butyl p-Hydroxibenzoat, Domiphen Bromid, Natrium Propionat, Propylen Glykol und ihre Mischungen.
zusammen mit einer ausreichenden Menge anderer pharmazeutisch akzeptierbarer Trägerstoffe mit Eigenschaften zur Einstellung der Viskosität und Beeinflussung des Geschmacks.

2. Zusammensetzung übereinstimmend mit Anspruch 1, **dadurch gekennzeichnet, dass** Das pH-Regulierungsmittel ausgewählt wird aus der Gruppe, die sich aus anhydrischer Zitronensäure, Monohydrat-Zitronensäure, Milchsäure, Weinsäure, Fumarsäure, Malinsäure, ihren entsprechenden Alkalisalze und ihren Mischungen zusammensetzt.

3. Zusammensetzung übereinstimmend mit Anspruch 2, **dadurch gekennzeichnet, dass** das pH-Regulierungsmittel ausgewählt ist aus der Gruppe, die sich aus anhydrischer Zitronensäure, Monohydrat-Zitronensäure, Natriumzitrat, Kaliumzitrat und ihren Mischungen zusammensetzt.

4. Zusammensetzung nach irgendeinem der Ansprüche 1 bis 3, **dadurch gekennzeichnet, dass** die Menge des Konsiervierungsmittels zwischen 0,1 und 0,2 Gew.-% pro Volumen liegt und dieses ausgewählt ist aus der Gruppe bestehend aus Benzoesäure, Natriumbenzoat, Kaliumsorbat, Sorbinsäure und ihren Mischungen.

5. Zusammensetzung nach einem der vorhergehenden Ansprüche, **dadurch gekennzeichnet, dass** sie zwischen 0,1 und 1 Gew.-% pro Volumen eines Mittels zur Einstellung der Viskosität enthält, das ausgewählt ist aus der Gruppe bestehend aus Hypromellose, Hydroxipropyl-Zellulose, Hydroxiethyl-Zellulose, Hydroxipropyl-Methylzellulose, Xanthan, Guaran, Tracanthgummi, Bentonit, PropylenGlycol-Alginat, Natriumalginat, Providon, Magnesium und Aluminiumsilikat, Kaolin, Gelatin, kolloidales Siliziumdioxid und ihren Mischungen.

6. Zusammensetzung nach Anspruch 5, **dadurch gekennzeichnet, dass** das Mittel zur Einstellung der Viskosität ausgewählt ist aus der Gruppe bestehend aus Xanthan, Guaran, Hydroxipropyl-Zellulose, Hydroxiethyl-Zellulose, Hydroxipropyl-Methylzellulose und ihren Mischungen.

7. Zusammensetzung nach einem der vorhergehenden Ansprüche, **dadurch gekennzeichnet, dass** sie eine wirksame Menge von Benetzungsmitteln enthält, ausgewählt aus der Gruppe bestehend aus Glycerol, Propylen-Glykol, flüssige Polyethylen-Glykole, Sorbitol und ihren Mischungen.

8. Zusammensetzung nach einem der vorhergehenden Ansprüche, weiter **gekennzeichnet dadurch, dass** sie eine wirksame Menge von Geschmackvermittlern enthält, ausgewählt aus der Gruppe bestehend aus Saccharin, Thaumatin, Meltitolsirup, Acesulfam, Aspartam, Maltitol, Lactitol, Natriumzyklamat, Xylitol, Sorbitol und ihren Mischungen.

9. Zusammensetzung nach einem der vorhergehenden Ansprüche, **dadurch gekennzeichnet, dass** sie 0,0005 bis 0,010 Gew.-% pro Volumen an Thaumatin als Geschmackvermittler enthält.

10. Verfahren für die Herstellung einer wässrigen, flüssigen pharmazeutischen Zusammensetzung in Form einer Suspensión für orale Verabreichung von Ibuprofen als Wirkstoff, **gekennzeichnet dadurch, dass** es wie folgt abläuft:
a) Vorlage einer wässrigen Lösung mit dem Konsiervierungsmittel und der pH-Reglersubstanz.
b) Zugabe von Natrium-Saccharin, Natrium-Chlorid und anderen Geschmacksvermittlern.
c) Zugabe eines Mittels zur Einstellung der Viskosität.
d) Benetzung des Wirkstoffes durch Hinzufügen eines Benetzungsmittels.
e) Hinzugabe eines aktiven Netzprinzips, erhalten unter Punkt d), zu der Mischung von Punkt c).

11. Verfahren übereinstimmend mit Anspruch 10, **gekennzeichnet dadurch, dass** alle Komponenten bei Umgebungstemperatur hinzugefügt werden.

12. Verfahren nach einem der Ansprüche 10 oder 11, **gekennzeichnet dadurch, dass** die Menge des im Schritt a) hinzugefügten Konservierunasmittels zwischen 0,1 und 0,2 Gew.-% pro Volumen liegt.

13. Verfahren nach einem der Ansprüche 10 bis 12, **gekennzeichnet dadurch, dass** die in Schritt b) hinzugefügte Menge an Natrium-Saccharin und Natrium-Chlorid zwischen je 0,01 und 0,5 Gew.-% und 0,1 und 2 Gew.-% pro Volumen liegt.

14. Verfahren nach einem der Ansprüche 10 bis 13, **gekennzeichnet dadurch, dass** die Menge des pH-Reglers zwischen 0,1 und 2 Gew.-% pro Volumen liegt, so dass der pH-Wert zwischen 3,8 und 4,8 eingestellt wird.

## Revendications

1. Composition pharmaceutique liquide à base d'eau sous forme de suspension pour l'administration par voie orale d'ibuprofen comme principe actif, **caractérisée en ce que** ladite composition contient :
a) de 0,8 % à 6 % en poids par volume d'ibuprofen
b) de 0,1 % à 2 % en poids par volume de chlorure de sodium ;
c) de 0,01 % à 0,5 % en poids par volume de saccharine de sodium ;
d) de 0,1 % à 2 % en poids par volume d'un ou plusieurs agents régulateurs du pH dans des quantités suffisantes pour ajuster le pH entre 3.8 et 4.8 ;
e) de 0,01 % à 0,5 % en poids par volume d'un conservateur choisi parmi le groupe composé de benzoate de sodium, acide benzoïque, sorbate de potassium, acide sorbique, méthyle p-hydroxybenzoate, éthyle p-hydroxybenzoate, propyle p-hydroxybenzoate, butyle p-hydroxybenzoate, méthyle p-hydroxybenzoate de sodium, éthyle p-hydroxybenzoate de sodium, propyle p-hydroxybenzoate de sodium, butyle p-hydroxybenzoate de sodium, bromure de domiphen, propionate de sodium, glycol de propylène et leurs mélanges.
conjointement avec une quantité appropriée d'autres excipients pharmaceutiquement acceptables ayant des caractéristiques de viscosification et masquage de goût.

2. Composition selon la Revendication 1, **caractérisée en ce que** l'agent régulateur de pH est choisi parmi le groupe composé d'acide citrique anhydre, acide citrique monohydrate, acide lactique, acide tartarique, acide fumarique, acide malique, leurs sels alcalins correspondants et leurs mélanges.

3. Composition selon la Revendication 2, **caractérisée en ce que** l'agent régulateur de pH est choisi parmi le groupe composé d'acide citrique anhydre, acide citrique monohydrate, citrate de sodium, citrate de potassium et leurs mélanges.

4. Composition selon n'importe laquelle des revendications 1 à 3, **caractérisée en ce que** la quantité de conservateur varie entre 0,1 % et 0,2 % en poids par volume et que ce dernier est choisi parmi le groupe composé d'acide benzoïque, benzoate de sodium, sorbate de potassium, acide sorbique et leurs mélanges.

5. Composition selon n'importe laquelle des revendications précédentes, **caractérisée en ce qu'**il y a entre 0,1 % et 1 % en poids par volume d'un viscosifieur choisi parmi le groupe composé d'hypromellose, cellulose d'hydroxypropyle, cellulose d'hydroxyéthyle, méthylcellulose d'hydroxypropyle, gomme xanthane, gomme de guar, gomme adragante, bentonite, alginate de glycol de propylène, alginate de sodium, povidone, silicate de magnésium et d'aluminium, kaolin, gélatine, dioxyde de silice colloïdal et leurs mélanges.

6. Composition selon la Revendication 5, **caractérisée en ce que** le viscosifieur est choisi parmi le groupe composé de gomme adragante, gomme de guar, cellulose d'hydroxypropyle, cellulose d'hydroxyéthyle, methylcellulose d'hydroxypropyle et leurs mélanges.

7. Composition selon n'importe laquelle des revendications précédentes, **caractérisée en ce qu'**il y a une quantité effective d'un agent humidifiant choisi parmi le groupe composé de glycérol, glycol de propylène, glycols de polyéthylène liquide, sorbitol et leurs mélanges.

8. Composition selon n'importe laquelle des revendications précédentes, **caractérisée en ce qu'**il y a une quantité effective d'un agent de masquage de goût choisi parmi le groupe composé de saccharine, thaumatine, sirop de maltitol, potassium d'acésulfame, aspartame, maltitol, lactitol, cyclamate de sodium, xylitol, sorbitol et leurs combinaisons.

9. Composition selon n'importe laquelle des revendications précédentes, **caractérisée en ce qu'**il y a entre 0,0005 % et 0,010 % en poids par volume de thaumatine en tant qu'agent de masquage de goût.

10. Processus pour la préparation d'une composition pharmaceutique liquide à base d'eau sous forme de suspension pour l'administration par voie orale d'ibuprofen comme principe actif, **caractérisée en ce qu'**elle consiste en :
a) la préparation d'une solution aqueuse avec le conservateur et les agents régulateurs du pH ;
b) l'addition de saccharine de sodium, chlorure de sodium et autres agents de masquage de goût ;
c) l'addition d'agents viscosifiants ;
d) l'humidification du principe actif moyennant l'incorporation d'un agent humidifiant ;
e) l'incorporation d'un principe humidifiant actif, obtenu au point d), dans le mélange obtenu au point c).

11. Processus selon la Revendication 10, **caractérisé en ce que** tous les composants sont ajoutés à température ambiante.

12. Processus selon la Revendication 10 ou 11, **caractérisé en ce que** la quantité de conservateur ajouté à l'étape a) varie de 0,1 % à 0,2 % en poids par volume.

13. Processus selon n'importe laquelle des Revendications 10 à 12, **caractérisé en ce que** la quantité de saccharine de sodium et de chlorure de sodium ajoutée à l'étape b) se trouve respectivement entre 0,01 % et 0,5 %, et 0,1 % et 2 % en poids par volume.

14. Processus selon n'importe laquelle des Revendications 10 à 13, **caractérisé en ce que** la quantité d'agents régulateurs du pH se trouve entre 0,1 % et 2 % en poids par volume, pour que le pH soit ajusté entre 3.8 et 4.8.
